# EUROPEAN PATENT APPLICATION

(11) **EP 4 810 046 A1**
(43) Date of publication of application: **23.09.2026**
(21) Application number: 24890668.7
(22) Date of filing: 12.11.2024
(51) Int. Cl.: A61K 31/56, A61K 9/06, A61P 25/00, A61P 25/08

(54) **USE OF PENTACYCLIC TRITERPENE COMPOUND TARGETING HYDROXYSTEROID 17-B DEHYDROGENASE 4**

(30) Priority: 13.11.2023 CN 202311510213
(71) Applicant: Jiangsu Bcy Pharm. Co., Ltd, Suzhou, Jiangsu 215123 (CN)
(72) Inventor: WANG, Honglin, Suzhou, Jiangsu 215123 (CN); GAO, Min, Suzhou, Jiangsu 215123 (CN)
(74) Representative: V.O.
(86) International application number: PCT/CN2024/131585
(87) International publication number: WO 2025/103313

(57) **Abstract**

Provided is a use of a pentacyclic triterpene compound targeting hydroxysteroid 17-β dehydrogenase 4. The pentacyclic triterpene compound can effectively relieve or treat central nervous diseases (inflammatory diseases), including degenerative diseases of the central nervous system or chronic central inflammatory diseases. The pentacyclic triterpene compound can maintain the structural stability or activity of hydroxysteroid 17-β dehydrogenase and maintain the stability or activity of peroxisome, thereby relieving or treating central nervous inflammation. The pentacyclic triterpene compound can target hydroxysteroid 17-β dehydrogenase 4, inhibit neuroglial cell overactivation related neuroinflammation, relieve neuron damage or death rate, and improve the cell metabolism microenvironment of the central nervous system.

## Description

The disclosure claims priority to the patent application with application number CN 202311510213.4, submitted on November 13th, 2023, the entire contents of which are hereby incorporated by reference.

### TECHNICAL FIELD

The disclosure belongs to the field of medicine; more specifically, it relates to use of a class of pentacyclic triterpene compounds targeting hydroxysteroid 17-β dehydrogenase 4.

### BACKGROUND OF THE DISCLOSURE

Some degenerative diseases of the central nervous system (CNS), represented by Alzheimer's disease (AD), manifest as a progressive and comprehensive decline in cognitive function, behavioral capacity, and mental status, with gradual loss of self-care ability and eventual death of patients, and this disease is considered one of the most serious global health and social crises of the 21st century. The annual treatment cost for AD patients is high, and the burden of care services and medical insurance cannot be ignored, imposing a heavy economic burden on pati ents and society. As the current trend of population aging further intensifies, the incidence of age-related cognitive disorders represented by AD increases year by year, making them major diseases and social issues that endanger public health. Given the growing market demand for AD treatments mismatched with current clinical drug production capacity, and the fact that AD drug development pipelines are narrow and have extremely high failure rates, it is imperative to comprehensively explore the mechanisms underlying the onset and progression of Alzheimer's disease and to identify new therapeutic targets and drug development pathways.

As CNS degenerative diseases, several diseases such as Parkinson's disease (PD) and dementia with Lewy bodies (DLB) feature Lewy bodies (LBs), which are rich in aggregated forms of α-synuclein (α-syn). α-Syn is a 14 kDa unstructured protein primarily produced in neurons. Under pathological conditions, the monomeric form of the protein gradually forms oligomeric structures and insoluble fibrillar assemblies, accumulating intracellularly as LBs. Overexpression or mutation of α-syn leads to progressive deficits and loss of dopaminergic neurons in the substantia nigra. Studies have shown that α-syn pathology can spread from cell to cell, thereby driving disease progression. α-Syn and mitochondria within microglia can be transferred and degraded via intercellular membrane protrusions, which is a possible mechanism of cell -to-cell propagation.

Multiple sclerosis (MS) is an incurable inflammatory autoimmune disease of the CNS that affects millions of people worldwide. MS is a chronic demyelinating disease of the CNS, and its onset and progression are driven by a combination of immune dysregulation, genetic susceptibility, and environmental factors. Activation of microglia and astrocytes is a key participant in the immunopathology of MS, playing specific roles depending on the anatomical location and stage of the disease, and controlling demyelination and neurodegeneration. Laura A. Pasquini, et al suggest that the worsening of myelin debris phagocytosis after microglial depletion reflects the central role of microglia in demyelination. Inadequate phagocytosis subsequently hinders remyelination, particularly in myelin-rich areas, and leads to neurodegeneration. Astrocytes are also involved in myelin uptake, especially as an early response to injury that ultimately triggers immune cell recruitment. This early response may positively or negatively affect lesion pathology, specifically depending on the inflammatory environment, which itself is altered by microglial depletion. Overall, microglial depletion may disrupt the inflammatory landscape of demyelinating lesions, promoting beneficial or detrimental responses of astrocytes and oligodendrocytes, thereby affecting neurodegeneration. Thus, the importance of modulating glia-mediated neuro inflammation as a drug target for relapsing-remitting MS is elucidated, and there is great potential for drugs that regulate and maintain glial homeostasis in treating multiple sclerosis and other diseases.

### SUMMARY OF DISCLOSURE

The object of the disclosure is to provide preparation and use of pentacyclic triterpene compounds targeting hydroxysteroid 17-β dehydrogenase 4.

In the first aspect, the present disclosure provides use of a compound as shown in formula (I), or an isomer, solvate, prodrug, or pharmaceutically acceptable salt thereof, in the preparation of a pharmaceutical composition for relieving or treating central nervous inflammation; 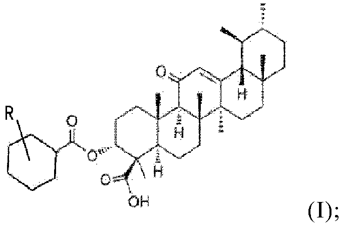 wherein R is independently selected from: hydrogen, hydroxy, C1-C4 alkyl, C2-C4 alkenyl, C2-C4 alkynyl, halogen.

In one or more embodiments, the compound as shown in formula (I), or an isomer, solvate, prodrug, or pharmaceutically acceptable salt thereof, can target and bind to hydroxysteroid 17-β dehydrogenase 4, maintain the structural stability or activity of hydroxysteroid 17-β dehydrogenase and maintain the stability or activity of peroxisomes, thereby relieving or treating central nervous inflammation

In one or more embodiments, the compound as shown in formula (I), or an isomer, solvate, prodrug, or pharmaceutically acceptable salt thereof, can target hydroxysteroid 17-β dehydrogenase 4, inhibit neuroglial cell (comprising microglia) overactivation related neuroinflammation, relieve neuron damage or death rate, or improve the cell metabolism microenvironment of the central nervous system.

In one or more embodiments, the central nervous inflammation is central nervous inflammation with dysfunction of hydroxysteroid 17-β dehydrogenase 4 (comprising decreased or absent expression, absent or decreased activity, decreased stability, etc.).

In one or more embodiments, the central nervous inflammation comprises: degenerative diseases of the central nervous system or chronic central inflammatory diseases.

In one or more embodiments, the degenerative diseases of the central nervous system comprise: Parkinson's disease (PD), amyotrophic lateral sclerosis (ALS), Alzheimer's disease (AD), Huntington's disease (HD), or a disease of reduced learning ability or memory.

In another aspect, the present disclosure provides use of a compound as shown in formula (I), or an isomer, solvate, prodrug, or pharmaceutically acceptable salt thereof, in the preparation of a pharmaceutical composition for maintaining the structural stability or activity of hydroxysteroid 17-β dehydrogenase 4 (MFE-2), or maintaining the stability or activity of peroxisomes.

In another aspect, the present disclosure provides use of a compound as shown in formula (I), or an isomer, solvate, prodrug, or pharmaceutically acceptable salt thereof, in the preparation of a pharmaceutical composition for inhibiting neuroglial cell overactivation related neuroinflammation, relieving neuron damage or death rate, or improving the cell metabolism microenvironment of the central nervous system.

In one or more embodiments, the pharmaceutical composition further comprises donepezil; preferably, the ratio of the compound as shown in formula (I) to donepezil is from 10:1 to 60:1, preferably from 15:1 to 50:1, more preferably from 20:1 to 45:1 (such as 25:1, 30:1, 33:1, 35:1 or 40:1).

In one or more embodiments, the compound as shown in formula (I) is a compound with the structure as shown in formula (II).

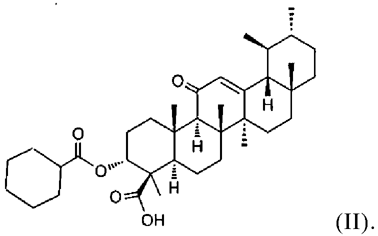

Wherein, the English name of the compound as shown in formula (II) is 3-o-α-cyclohexanoyl-11-keto-β-boswellic acid, abbreviated as CKBA (molecular formula: C37H56O5, molecular weight: 581).

In another aspect, the present disclosure provides a composition (comprising a cell culture medium) for relieving or treating central nervous inflammation, comprising: a compound as shown in formula (I), or an isomer, solvate, prodrug, or pharmaceutically acceptable salt thereof; preferably, the pharmaceutical composition further comprises donepezil; and preferably, the ratio (weight ratio) of the compound as shown in formula (I) to donepezil is from 10:1 to 60:1, preferably from 15:1 to 50:1, more preferably from 20:1 to 45:1 (such as 25:1, 30:1, 33:1, 35:1 or 40:1).

In one or more embodiments, the compound as shown in formula (I), or an isomer, solvate, prodrug, or pharmaceutically acceptable salt thereof, serves as the main active ingredient (active component) in the composition (comprising a pharmaceutical composition). Preferably, donepezil also serves as a main active ingredient.

In one or more embodiments, the compound as shown in formula (I), or an isomer, solvate, prodrug, or pharmaceutically acceptable salt thereof, serves as the sole active ingredient (active component) in the composition (comprising a pharmaceutical composition). Alternatively, the compound as shown in formula (I), or an isomer, solvate, prodrug, or pharmaceutically acceptable salt thereof, together with donepezil, serve as the only active ingredients (active components).

In one or more embodiments, the compound as shown in formula (I), or an isomer, solvate, prodrug, or pharmaceutically acceptable salt thereof, can also be used in combination with other active ingredients, each exerting its independent effect.

In one or more embodiments, the compound as shown in formula (I) is prepared by a method comprising: using 11-keto-β-acetylboswellic acid as a starting material, and replacing its AcO group with 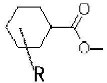 group.

In one or more embodiments, the compound as shown in formula (I) is prepared by a method comprising:
(i) reacting 11-keto-β-acetylboswellic acid with a base to obtain 11-keto-β-boswellic acid; and
(ii) reacting 11-keto-β-boswellic acid with cyclohexanecarbonyl chloride to obtain the compound as shown in formula (I).

In one or more embodiments, the compound as shown in formula (I) is formulated with a pharmaceutically acceptable excipient to form a pharmaceutical composition; preferably, the compound as shown in formula (I) is in a systemic/internal dosage form.

In one or more embodiments, the composition is an oral dosage form, comprising a suspension or a tablet for administration.

In one or more embodiments, a method for preparing an oral dosage form of the compound as shown in formula (I) comprises: (1) preparing a suspension of the compound as shown in formula (I): using a 0.1-1% (w/v) sodium carboxymethylcellulose solution to prepare the powder suspension, at a final concentration of 10-20 mg/mL, for oral administration at a dose of 100±50 mg per kg of body weight.

In one or more embodiments, a method for preparing an oral dosage form of the compound of formula (I) comprises: (1) preparing a tablet of the compound as shown in formula (I): selecting diluents such as microcrystalline cellulose 101, hydroxypropyl methylcellulose, and/or starch; selecting three disintegrants: low-substituted hydroxypropyl cellulose, crospovidone, and/or croscarmellose sodium; using disintegration time and dissolution rate of the tablet as indicators to determine the types and amounts of filler and disintegrant; (2) mixing the prepared powder uniformly with the diluent and disintegrant, compressing into large tablets using a single-punch tablet press, then crushing the large tablets in a mortar to form granules, finally adding the lubricant magnesium stearate to the granules, sieving (e.g., through a 30-mesh sieve) to granulate, and directly compressing into tablets using a single-punch tablet press to obtain the tablets.

In one or more embodiments, the compound as shown in formula (I) is dissolved in methanol/DMSO, efficiently crossing the blood-brain barrier; preferably, a QTRAP 6500 plus (SCIEX) mass spectrometer is used to determine the MS1 and MS2 information and corresponding mass spectrometric parameters of the compound, thereby determining the quantitative ion pair, and then a quantitative method is established using a compound standard test to accurately quantify the content and relative levels of the compound in brain tissue and plasma.

In another aspect, the present disclosure provides a kit or a reagent kit for relieving or treating central nervous system inflammation, comprising the pharmaceutical composition described above.

In another aspect, the present disclosure provides a method for relieving or treating central nervous inflammation, the method comprising: administering an effective amount of the pharmaceutical composition of the present disclosure to a subject in need thereof.

Other aspects of the disclosure will be apparent to those skilled in the art based on the disclosure herein.

### BRIEF DESCRIPTION OF DRAWINGS

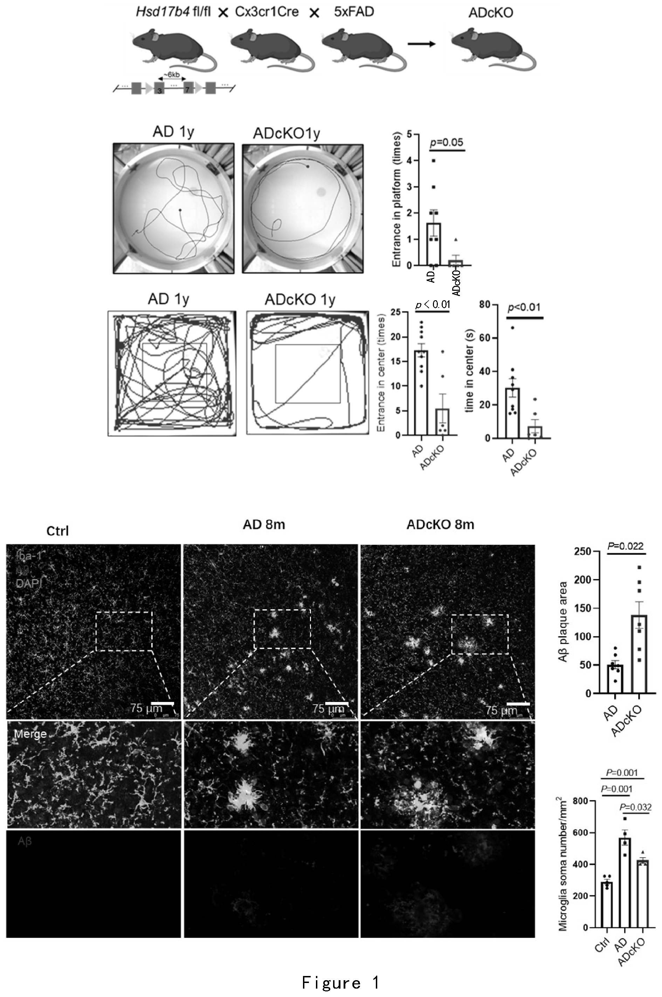

Figure 1. MFE-2 is a key regulatory target in AD. Conditional knockout of MFE-2 in microglia of AD mice (MFE-2^{cKO}5xFAD mice, upper panel) was performed, followed by behavioral tests (middle panel), Aβ and microglial staining. Immunofluorescence staining revealed that in the brains of MFE-2 conditional knockout AD mice, the morphology of microglia in the early stage was significantly abnormal, and the density of branched synaptic protrusions was reduced, suggesting impaired immune surveillance and neural support functions. By the time the mice survived to 8 months of age (8m), the number of Aβ plaques in the hippocampal region of the brain was significantly increased (lower panel), indicating that MFE-2 deficiency causes immune inflammatory dysfunction and exacerbates Aβ plaque deposition in brain tissue.

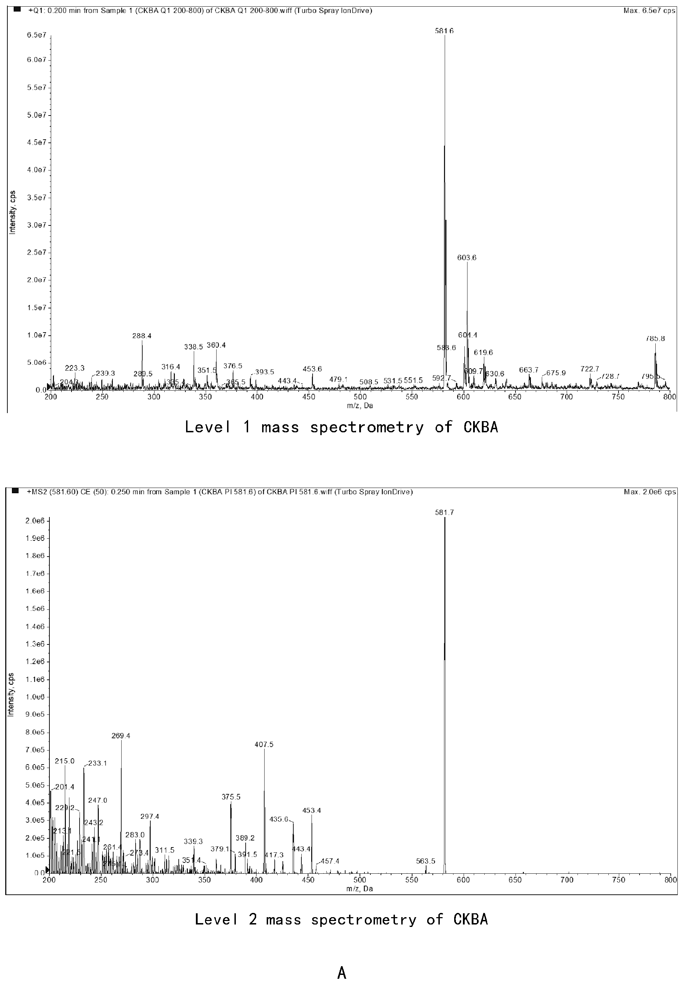

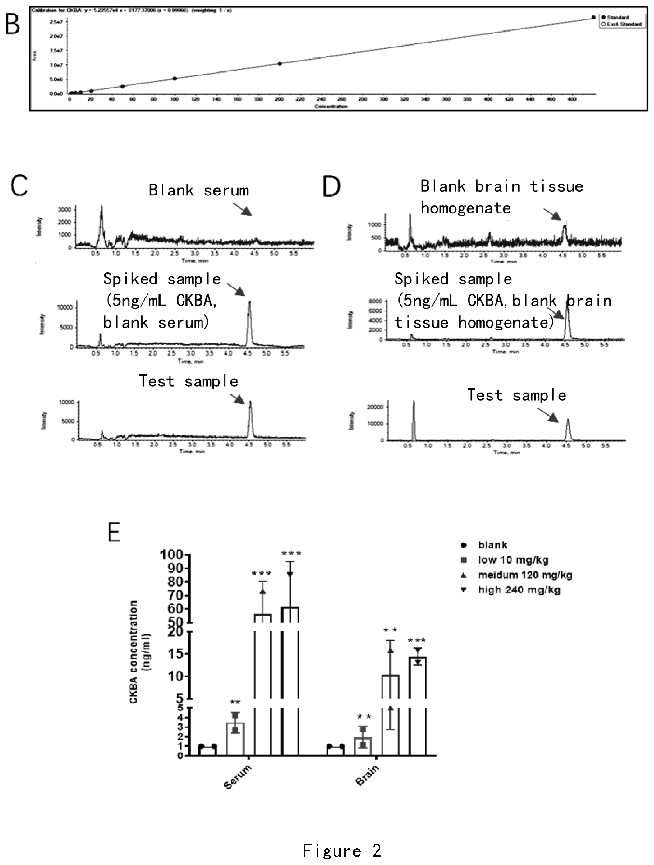

Figure 2. CKBA administered by oral gavage efficiently penetrates the blood-brain barrier. Mass spectrometry was used to evaluate the blood-brain barrier permeability of CKBA. Normal mice were administered CKBA by oral gavage at 2.4 mg/mouse (100 mg/kg). One hour after administration, the mice were perfused, and fresh brain tissue and serum were collected for mass spectrometry analysis. It was found that CKBA could effectively cross the blood-brain barrier.

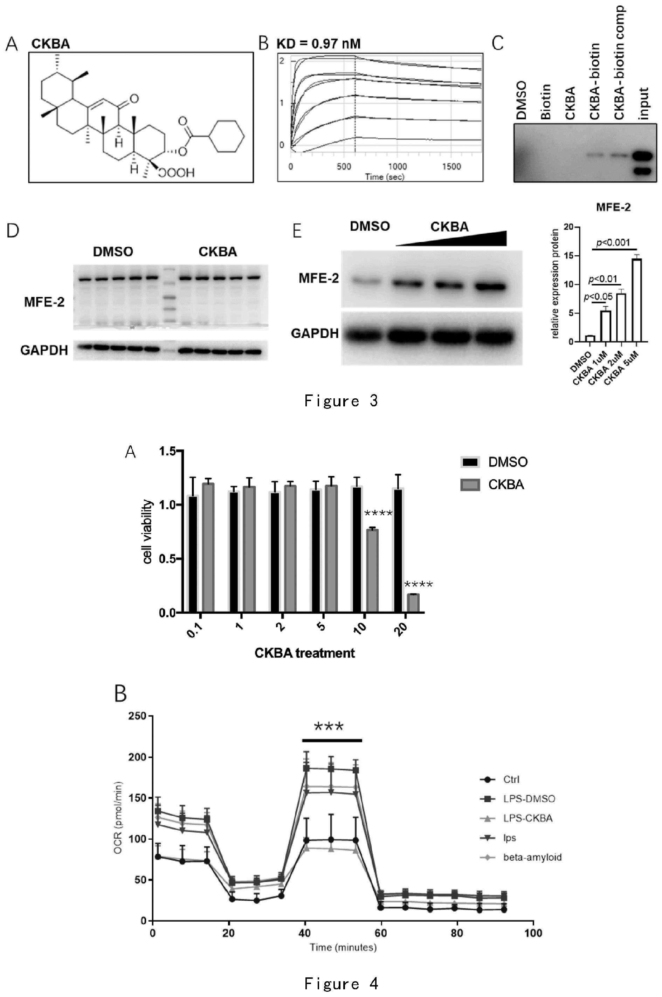

Figure 3. CKBA targets and binds to MFE-2 protein with high affinity. Studies on the anti-inflammatory mechanism of CKBA and its target revealed that CKBA has a very strong affinity for MFE-2.
Figure 4. CKBA inhibits inflammatory activation of microglia. In vitro cell experiments were conducted to explore the effect of CKBA on the inflammatory activation of microglia. After CKBA was added to LPS activated BV2 cells for 24 hours, it was found that CKBA significantly inhibited the expansion of inflammatory microglia, maintained the structural stability of MFE-2 and thereby inhibited protein degradation, maintained the stability of peroxisomes, and exerted an anti-inflammatory effect (panel A). Panel B shows quantitative analysis of intracellular mitochondrial stress levels.

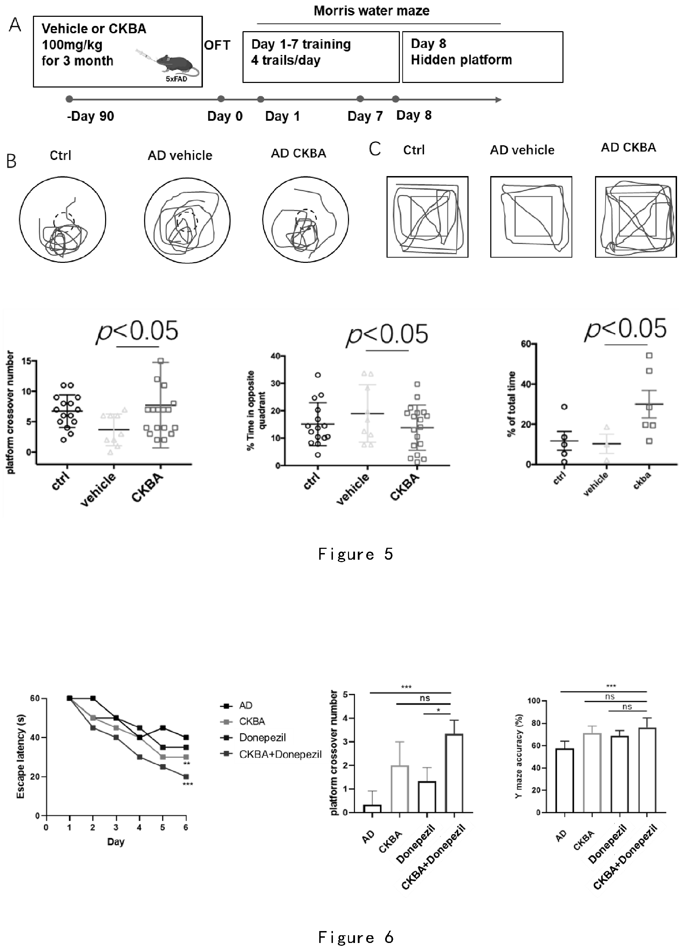

Figure 5. CKBA treatment effectively alleviates pathological changes in an AD mouse model. AD mice were treated with CKBA by oral gavage for in vivo intervention studies, with continuous intervention for 3 months.
Figure 6. The therapeutic effect of CKBA combined with donepezil is significantly superior to that of CKBA or donepezil monotherapy. AD mice were treated with CKBA combined with donepezil by oral gavage for in vivo intervention studies. After continuous intervention for 3 months, the degree of deterioration of higher central functions such as learning and memory in AD mice was analyzed. The Morris water maze test was used to measure escape latency and platform crossover number, and the Y-maze test was used to assess the spatial memory and learning ability of the mice.

### DETAILED DESCRIPTION

The inventors have dedicated efforts to exploring novel molecular therapeutic targets of the pentacyclic triterpene compounds as shown in formula (I). Through in-depth research and analysis, a high-affinity functional target of the pentacyclic triterpene compounds as shown in formula (I), namely hydroxysteroid 17-β dehydrogenase 4 (MFE-2), has been identified. Meanwhile, the inventors' analyses show that the pentacyclic triterpene compound as shown in formula (I) can effectively relieve or treat central nervous diseases (inflammatory diseases), including degenerative diseases of the central nervous system or chronic central inflammatory diseases.

### Terms

A person skilled in the art will understand the terms. Specifically, as used herein, the term "alkyl" refers to a straight or branched chain saturated aliphatic hydrocarbon group comprising 1 to 4 carbon atoms (preferably 1 to 2 carbon atoms). For example, alkyl comprises, but is not limited to, methyl, ethyl, n-propyl, isopropyl, n-butyl, isobutyl, and tert-butyl.

As used herein, the term "alkenyl" comprises straight and branched chain hydrocarbon groups comprising at least one carbon-carbon double bond and 2 to 4 carbon atoms (preferably 2 to 3 carbon atoms).

As used herein, the term "alkynyl" comprises straight and branched chain hydrocarbon groups comprising at least one carbon-carbon triple bond and 2 to 4 carbon atoms (preferably 2 to 3 carbon atoms).

As used herein, the term "halogen" refers to F, Cl, Br, or I.

As used herein, the term "isomer" comprises: geometric isomers, enantiomers, diastereomers (e.g., cis-trans isomers, conformational isomers).

As used herein, the 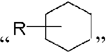 is well known to those skilled in the art, indicating that the group R may be substituted at any one or more substitutable positions on the ring. Moreover, at different substitution positions, the choice of R may be different.

As used herein, the term "solvate" refers to a compound that carries solvent molecules; for example, the solvate may be a hydrate.

In the present disclosure, the term "comprising" indicates that various components may be used together in the mixture or composition of the present disclosure. Thus, the terms "consisting essentially of" and "consisting of" are encompassed within the term "comprising".

In the present disclosure, a "pharmaceutically acceptable" component is a substance that is suitable for use in humans and/or animals without excessive adverse side effects (such as toxicity, irritation, and allergic reactions), i.e., having a reasonable benefit/risk ratio.

In the present disclosure, "pharmaceutically acceptable carrier" refers to a pharmaceutically or food-acceptable solvent, suspending agent, or excipient used to deliver the compound as shown in formula (I), isomer, solvate, prodrug, or pharmaceutically acceptable salt thereof of the present disclosure to an animal or human. The carrier may be liquid or solid.

As used in the present disclosure, the "modulation" comprises "up-regulation" and "down-regulation", wherein "up-regulation" comprises, but is not limited to: promotion, increase, elevation, enhancement, etc.; and "down-regulation" comprises, but is not limited to: reduction, inhibition, antagonism, blockade, blocking, etc.

### Molecular Target

MFE-2, encoded by the *HSD17B4* gene, is a bifunctional enzyme involved in the peroxisomal β-oxidation pathway of fatty acids. It also acts as a catalyst for the formation of 3-ketoacyl-CoA intermediates from straight-chain and 2-methyl-branched-chain fatty acids. Defects in this gene that affect peroxisomal fatty acid β-oxidation activity directly lead to D-bifunctional protein deficiency (DBPD), causing severe central nervous dysfunction.

Microglia are the most abundant immune cell type in the central nervous system, and microglia-mediated neuroinflammation is a common, critical pathological feature and risk factor for various central nervous system degenerative diseases. The inventors found that in an AD mouse model, the expression level of MFE-2 in microglia is significantly decreased, and the number of peroxisomes is markedly reduced, consistent with the increase in microglial activation and elevated levels of inflammatory cytokines such as IL-6 and IL-1β in the brain. Further, after specific knockout of MFE-2 in microglia of 5xFAD mice, compared with 5xFAD mice, the knockout mice exhibited abnormal microglial morphology, decreased immune surveillance function, and a persistently hyperactivated phenotype in the brain at an earlier age, along with significantly impaired behavioral functions such as memory. Intervention with a pentacyclic triterpene compound (preferably CKBA) effectively maintained the level of MFE-2 protein in microglia, maintained intracellular homeostasis and redox balance, and slowed down the deterioration of learning and memory functions in the individual's brain. The research results strongly suggest that loss of MFE-2 function and lipid metabolism disorders can disrupt microglial immune homeostasis, further leading to microglial dysfunction and exacerbating the onset and progression of neurodegenerative diseases under aging stress.

Therefore, microglial dysfunction is a key upstream factor of central immune dysregulation and neuroinflammation in the process of central nervous system degeneration. Microglial lipid metabolism is an important intervention target for regulating microglial functional phenotype, alleviating neuroinflammation, and slowing disease progression. In particular, the inventors have confirmed that a reduction in MFE-2 protein level leads to severe central nervous system disorders, especially when MFE-2 deficiency in microglia results in persistent central nervous system inflammatory responses, exacerbating neurodegeneration and causing severe cognitive impairment. However, there is currently no targeted drug against MFE-2.

The present disclosure discloses for the first time that the pentacyclic triterpene compound (preferably CKBA) binds to MFE-2 with high affinity and stabilizes the level of MFE-2 protein in cells. The compound acts on microglia, effectively controlling the level of central inflammation by stabilizing MFE-2 expression, thereby ultimately alleviating the progression of neurodegenerative diseases. Therefore, CKBA, by targeting microglial MFE-2 to inhibit central inflammation, provides a universal solution for the central neuroimmune pathology of neurodegenerative diseases such as AD, PD, and MS, slows neurodegeneration, and thus is expected to effectively improve and enhance the health and quality of life of the aging population.

The CKBA can be safely and effectively administered systemically to individuals in need, in particular, it can efficiently cross the blood-brain barrier, then target intracellular MFE-2 protein, maintain normal intracellular metabolic functions, stabilize the glial cell metabolic microenvironment, inhibit excessive activation, significantly reduce the levels of inflammatory cytokines in neurodegenerative diseases, suppress central nervous system inflammation, and ultimately increase neuronal survival and reduce the accumulation of toxic proteins in the central nervous system. Therefore, it can be used in the preparation of drugs for treating neurodegenerative diseases. It has broad application prospects, and effectively solves the problems of scarcity of existing central inflammatory immunomodulatory drugs, insufficient efficacy, and low safety.

The pentacyclic triterpene compound can efficiently penetrate the blood-brain barrier and exhibits excellent activity by directly binding with high affinity to microglial MFE-2 protein, thereby acting on MFE-2 to maintain mitochondrial metabolic balance and significantly inhibit central nervous system inflammation associated with persistent microglial activation.

In a preferable embodiment, the "central nervous system inflammation" refers to a chronic, persistent, high inflammatory state within the central nervous system mediated by microglial activation, as well as other mechanisms involving microglia-astrocyte interaction in neuroinflammation. Microglia, as resident immune cells in the central nervous system, mediate neuroinflammation and play important roles in various physiological and pathological conditions. After neuronal injury, microglia rapidly initiate an inflammatory response; astrocytes and microglia regulate central nervous system inflammation by secreting various cytokines and inflammatory mediators.

### Pentacyclic Triterpene Compound

The present disclosure comprises the compound of formula (I), or an isomer, solvate, prodrug, or pharmaceutically acceptable salt thereof, as long as they also have the same or substantially the same function as the compound of formula (I). The "pharmaceutically acceptable salt" refers to a salt formed by the reaction of the compound with an inorganic acid, an organic acid, an alkali metal, an alkaline earth metal, or the like. These salts comprise (but are not limited to): (1) salts formed with inorganic acids, such as hydrochloric acid, sulfuric acid, nitric acid, phosphoric acid; (2) salts formed with organic acids, such as acetic acid, oxalic acid, succinic acid, tartaric acid, methanesulfonic acid, maleic acid, or arginine. Other salts comprise salts formed with alkali metals or alkaline earth metals (such as sodium, potassium, calcium, or magnesium), in the form of esters, carbamates, or other conventional "prodrugs". The compounds have one or more asymmetric centers. Therefore, these compounds may exist as racemic mixtures, individual enantiomers, individual diastereomers, diastereomeric mixtures, or cis or trans isomers.

The "prodrug of the compound" refers to a precursor of the compound that, when administered by an appropriate method, undergoes metabolism or chemical reaction in the patient's body to be converted into a compound of structural formula (I), or a salt or solution composed of a compound of chemical structural formula (I).

As a preferable embodiment of the present disclosure, the compound has the structure as shown in formula (II). The English name of the compound as shown in formula (II) is 3-o-α-cyclohexanoyl-11-keto-β-boswellic acid, abbreviated as CKBA. Its molecular formula is C₃₇H₅₆O₅ and its molecular weight is 581.

A person skilled in the art will understand that, once the structure of the compound of the present disclosure is known, the compound of the present disclosure can be obtained by various methods well known in the art, using known raw materials, such as chemical synthesis or extraction from organisms (e.g., animals or plants), and these methods are all comprised within the present disclosure.

For example, a method for preparing the compound as shown in formula (I) of the present disclosure comprises: using 11-keto-β-acetylboswellic acid as a starting material, and replacing its AcO- group with a 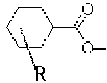 group. More preferably, the steps of preparation comprise: (i) using 11-keto-β-acetylboswellic acid (AKBA) as a starting material, reacting it with a base (e.g., KOH) to obtain 11-keto-β-boswellic acid; and (ii) reacting 11-keto-β-boswellic acid with cyclohexanecarbonyl chloride to obtain the compound of formula (I). Other methods for preparing the compound of formula (I) are also comprised within the present disclosure, for example, using 11-keto-β-boswellic acid (KBA) as a starting material and reacting it directly with cyclohexanecarbonyl chloride to obtain the compound of formula (I).

The synthesized compound can be further purified by column chromatography, high performance liquid chromatography, or the like. In addition, further purification can also be carried out by crystallization methods. It should be understood that various purification methods can be applied in the present disclosure.

In a preferable embodiment, the present disclosure provides a method for preparing a pharmaceutical composition using the pentacyclic triterpene compound, comprising the following steps: (1) preparing the pentacyclic triterpene compound; (2) preparing a suspension of the pentacyclic triterpene compound by using a 0.1-1% (w/v) sodium carboxymethylcellulose solution to suspend the powder of the pentacyclic triterpene compound, at a final concentration of 10 -20 mg/mL. Preferably, for example, it is administered orally at a dose of 100 ± 50 mg per kg of body weight.

In a preferable embodiment of the present disclosure, a tablet of the pentacyclic triterpene compound is prepared: wherein, (1) a suspension of the pentacyclic triterpene compound is prepared by using a 0.1-1% (w/v) sodium carboxymethylcellulose solution to suspend the powder of the pentacyclic triterpene compound, at a final concentration of 10-20 mg/mL, for oral administration at a dose of 100±50 mg per kg of body weight; (2) for the tablet of the pentacyclic triterpene compound: three diluents are selected: microcrystalline cellulose 101, hydroxypropyl methylcellulose, and starch; three disintegrants are selected: low-substituted hydroxypropyl cellulose, crospovidone, and croscarmellose sodium. Using the disintegration time and dissolution rate of the tablet as indicators, the types and amounts of filler and disintegrant are determined; (3) the prepared powder of the pentacyclic triterpene compound is mixed uniformly with the diluents and disintegrants, compressed into large tablets using a single-punch tablet press, then the large tablets are crushed in a mortar to form granules, and finally the lubricant magnesium stearate is added to the granules, passed through a 30-mesh sieve for granulation, and directly compressed into tablets using a single-punch tablet press to obtain the pentacyclic triterpene compound tablets.

In a preferable embodiment, the present disclosure provides a pharmaceutical composition for treating central nervous system degenerative diseases, wherein the pharmaceutical composition comprising an effective amount of the pentacyclic triterpene compound and a pharmaceutically acceptable carrier.

In a preferable embodiment of the present disclosure, the pentacyclic triterpene compound is administered systemically, preferably orally, providing operational convenience for long-term drug control of the disease condition and therapeutic flexibility for adjusting dosage and treatment course, while also providing the possibility of dynamic monitoring of drug concentration and adverse reactions.

In a preferable embodiment of the present disclosure, the in vivo dosage of the compound is 50-150 mg/kg.

In a preferable embodiment of the present disclosure, the compound enters a subject in need thereof after oral gavage/oral administration/feeding/mixing with drinking water/mixing with food.

In a preferable embodiment of the present disclosure, the subject treated with the compound comprises humans and non-human mammals.

In a preferable embodiment of the present disclosure, the in vitro experimental intervention concentration of the compound is 1-10 µM.

In a preferable embodiment of the present disclosure, the compound is dissolved in DMSO to prepare a stock solution, then diluted according to different dilution gradients, and added to the cell culture medium for treatment.

In a preferable embodiment of the present disclosure, a 0.1-1% (w/v) sodium carboxymethylcellulose solution is used to prepare a suspension at a drug concentration of 15 mg/mL, administered by oral gavage, with an individual dosage of 50-150 mg/kg/day.

In a preferable embodiment of the present disclosure, the pentacyclic triterpene compound is formulated into the diet/feed and administered with meals. It should be understood that, according to the actual clinical situation, a physician may also make appropriate dosage changes.

In a preferable embodiment of the present disclosure, the pentacyclic triterpene compound is dissolved in DMSO/methanol, etc., and made into a suspension, and/or medicated feed, and/or oral tablets.

In a preferable embodiment of the present disclosure, a preferable highly lipophilic small molecule compound is provided, more preferably capable of effectively crossing the blood-brain barrier and accumulating in the target organ, the brain, to exert a therapeutic effect.

In a preferable embodiment of the present disclosure, the therapeutic target cell is a mammalian cell, and the mammal comprises humans and non-human mammals.

In a preferable embodiment of the present disclosure, the therapeutic target cell is selected from the group consisting of: glial cells (comprising microglia, astrocytes, oligodendrocytes, ependymal cells, radial glial cells, satellite cells, Schwann cells, enteric glial cells, etc.) and various types of neuronal cells, or a combination thereof.

In a preferable embodiment of the present disclosure, the compound or composition is used for treating aging/injury-related degenerative diseases.

In a preferable embodiment of the present disclosure, the compound or composition is used for treating neurodegenerative diseases.

In a preferable embodiment of the present disclosure, the compound or composition is used for inhibiting the level of central inflammation and/or promoting the clearance of neurotoxic proteins and/or neuronal survival, and alleviating the degree of decline in learning and memory functions of the brain.

In a preferable embodiment of the present disclosure, the central nervous system degenerative diseases comprise AD, MS, PD, and any age-related degenerative diseases of the central nervous system, accompanied by a long-term persistently activated central inflammatory response.

The pentacyclic triterpene compound disclosed in the present disclosure is simple to synthesize for oral dosage forms, with good stability, low cost, no immunogenicity, and can specifically inhibit microglial differentiation in an inflammatory environment, showing a remarkably significant therapeutic effect, and the disease is not prone to relapse after treatment with few side effects.

In some embodiments, the pentacyclic triterpene compound may be linked or combined with a functional molecule. For example, the functional molecule is a marker having a tracing function, comprising but not limited to fluorescent dyes, MRI contrast agents, radioactive imaging agents, magnetic particles, or chemical reagents having a coloring function. For example, the marker having a tracing function or functional small molecule may be fluorescein isothiocyanate (FITC).

In some embodiments, the functional molecule is a functional small molecule, comprising inorganic small molecules and organic small molecules, having a molecular weight of less than 1000 Daltons.

In some embodiments, the functional molecule is a formulation having a molecular cargo - loading function, comprising but not limited to liposomes, polymers, dendritic molecules, nano-packaging formulations, and the like.

The linkage between the small analytical compound CKBA of the present disclosure and the functional molecule may be covalent linkage or non-covalent linkage. It should be understood that any linkage that retains the activity of the small molecule compound and the function of the functional molecule may be comprised in the present disclosure. Covalent linkage usually connects two molecules by forming a covalent bond, while some non-covalent linkages (not forming a covalent bond), such as coupling, adsorption, binding, etc., may also be applied. As a preferable embodiment of the present disclosure, the polypeptide and the functional molecule are linked via a chemical bond; more preferably, the chemical bond is a peptide bond.

### Combination Therapy

The present disclosure provides a method of combination therapy, comprising a method of using the pentacyclic triterpene compound in combination with donepezil.

The present disclosure provides a use of the pentacyclic triterpene compound and donepezil (or a combination thereof) in the preparation of a mixture, a pharmaceutical composition, or a kit for alleviating or treating central nervous system inflammation.

During administration, the pentacyclic triterpene compound may be administered first, followed by donepezil; or the order may be reversed; or they may be administered simultaneously. It should be understood that various methods of administration are comprised within the present disclosure.

The present disclosure provides a mixture of small molecule compounds, comprising: the pentacyclic triterpene compound and donepezil as active components.

The present disclosure provides a pharmaceutical composition, comprising: (a) an effective amount of the pentacyclic triterpene compound; (b) an effective amount of donepezil; and (c) a pharmaceutically acceptable carrier or excipient.

The pharmaceutical composition or mixture of the present disclosure can be prepared into any conventional formulation form by conventional methods. The dosage forms can be varied, as long as they allow the active ingredients to effectively reach the mammalian body. For example, they can be selected from: injections, infusions, tablets, capsules, and pills. The active components may be present in suitable solid or liquid carriers or diluents.

The mixture or pharmaceutical composition of the present disclosure may also be stored in a sterile apparatus suitable for injection or infusion. Generally, in the pharmaceutical composition of the present disclosure, the pentacyclic triterpene compound and donepezil as active ingredients may comprise 0.01-20% of the total weight of the pharmaceutical composition, and the remainder may be a pharmaceutically acceptable carrier.

The effective dosages of the pentacyclic triterpene compound and donepezil used may vary depending on the mode of administration and the severity of the disease to be treated. When necessary, the pentacyclic triterpene compound and donepezil may also be administered in combination with other active ingredients or drugs.

The present disclosure also provides a kit for relieving or treating central nervous system inflammation, wherein the kit comprises: container 1, and the pentacyclic triterpene compound placed in container 1; and container 2, and donepezil placed in container 2.

Both the pentacyclic triterpene compound and donepezil are small molecule compounds. Therefore, the kit may also comprise a mixture of the pentacyclic triterpene compound and donepezil, wherein the contents of the pentacyclic triterpene compound and donepezil are as described above.

In addition, the kit may also comprise some auxiliary administration materials, such as syringes for injection, and the like.

Furthermore, the kit may also comprise an instruction manual, explaining the method of relieving or treating central nervous system inflammation using the combination therapy of the present disclosure.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Materials and Methods

### 1. In vivo and in vitro preparation of CKBA and its formulations

Systemic administration of CKBA compound or derivatives thereof was used. CKBA (molecular formula: C37H56O5, molecular weight: 580.85, solubility: lipophilic, soluble in methanol/DMSO, insoluble in water, self-synthesized) was used. 3-o-α-cyclohexanoyl-11-keto-β-boswellic acid (CKBA) is a small molecule compound obtained by structural modification and optimization using the active natural product molecule AKBA as a lead compound. The specific synthesis comprises using AKBA as a starting material, subjecting it to hydrolysis under alkaline conditions to obtain the KBA intermediate, followed by cyclohexanecarbonylation of KBA and purification to obtain the CKBA compound. The detailed synthetic route is as follows:

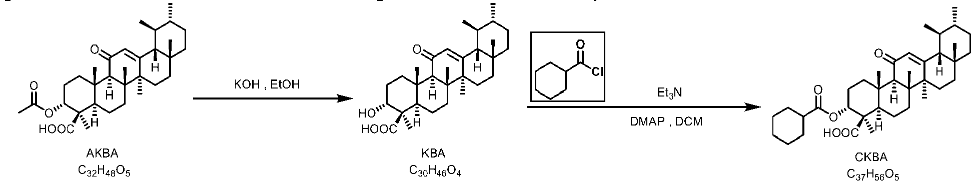

In vivo administration: A 0.1-1% (w/v) sodium carboxymethylcellulose solution was used to prepare a suspension at a drug concentration of 15 mg/mL, administered by oral gavage at an individual dosage of 50-150 mg/kg/day. Preferably, CKBA was formulated into the diet/feed and administered with meals at an individual dosage of 50-150 mg/kg/day.

Cell treatment: For cell treatment, CKBA was dissolved in DMSO to prepare a stock solution at a concentration of 5mM (1000×), stored at -20°C. For use, the stock solution was added to the cell culture medium at a dilution of 1:1000 and mixed well. The final working concentration was 5µM. Cells were treated for 24-48h and then collected for subsequent experiments.

### 2. Generation of MFE-2^{cKo}5xFAD, 5xFAD, MFE-2^{cKO} and MFE-2^{cKI}5xFAD mice to investigate the regulatory role of MFE-2 on microglial function

CRISPR-mediated homologous recombination was used to insert loxP sites in the mouse Hsd17b4 gene to generate Hsd17b4fl/fl mice. These mice were then bred with Cx3cr1Cre mice to obtain Cx3cr1CreHsd17b4fl/fl (MFE-2cKO) mice.

Hsd17b4fl/fl mice were bred with Cx3cr1Cre and 5xFAD mice (Alzheimer's disease mouse model) to obtain Cx3cr1CreHsd17b4^{fl/fl}5xFAD (MFE-2^{cKO}5xFAD) mice.

Littermate Hsd17b4^{fl/fl} (control) and Hsd17b4^{fl/fl} 5xFAD mice were used as controls with normal MFE-2 expression. Meanwhile, the CRISPR method was used to site-specifically insert an exogenous mHsd17b4 gene fragment into the mouse H11 locus. These Hsd17b4^{fl/fl} mice were then bred with Cx3cr1Cre and 5xFAD mice to obtain MFE-2^{cKI} and MFE-2^{cKI}5xFAD mice.

The regulatory role of MFE-2 on microglial homeostasis and inflammatory activation was observed at different time points during mouse development.

### 3. Immunofluorescence imaging analysis of microglia-mediated central nervous system inflammation under different animal model conditions

According to the progression of central nervous system inflammation and the distribution characteristics of microglia, MFE-2^{cKO}5xFAD, 5xFAD, MFE-2^{cKO} and control mice were euthanized at early (2 months), middle (4 months), and late (8 months) time points. After cardiac perfusion to thoroughly remove blood from brain tissue, the perfusate was switched to tissue fixative for whole-body perfusion fixation. Whole brain tissues were harvested, placed in fixative for 24 hours, washed with PBS, and dehydrated in a graded sucrose solution. The tissues were embedded in OCT, and 30-µm-thick tissue sections were prepared. Immunofluorescence staining was performed to analyze the expression levels of MFE-2, Iba-1, Cx3cr1, TNFα, F4/80 and IL-6.

Wherein, the immunofluorescence staining procedure mainly comprised incubation with 10% goat serum blocking solution, incubation with primary and secondary antibodies, and DAPI nuclear staining. After mounting, Leica SP8 laser scanning confocal microscopy was used t o capture the distribution and morphological characteristics of microglia; ImageJ software was used for microglial morphological analysis and quantitative analysis of intracellular fluorescence signal intensity.

### 4. Animal experiments and mass spectrometry method for CKBA penetration of the blood-brain barrier

Mass spectrometry standard solutions and standard curve for absolute quantification of CKBA: CKBA standard was diluted in methanol (10ng/mL, 1 ng/mL). A standard curve was prepared using CKBA standard solutions in methanol at concentrations of 0.5, 1, 2, 5, 10, 20, 50, 100, 200, and 500ng/mL. The above solutions were centrifuged (18800g, 15 min, 4°C), and 50µL of the supernatant was transferred to sample vials for mass spectrometry analysis. The spiked standard curve in blank serum was prepared as follows: 10µL of each concentration of CKBA working solution (prepared in methanol) was added to 90µL of blank serum and vortexed for 30s.

Sample preparation for detection: For double-blank serum samples, 10µL of methanol was added to 90µL of blank serum and vortexed for 30s. For actual serum samples, the spiked standard curve solutions in blank serum, blank serum samples, and actual serum samples were precipitated with 3 volumes of methanol, vortexed for 30s, and centrifuged (18800g, 15min, 4°C). Then, 50µL of the supernatant was transferred to sample vials for mass spectrometry analysis. For brain tissue homogenates, 1mL of methanol was added to each tube (6+2), thoroughly vortexed, and centrifuged (18800g, 15min, 4°C). The supernatant was taken and centrifuged again, and the resulting supernatant was reserved for use. The spiked standard curve in blank brain tissue homogenate was prepared as follows: 10µL of each concentration of CKBA working solution (prepared in methanol) was added to 90µL of blank brain tissue homogenate supernatant and vortexed for 30s. For blank brain tissue homogenate samples, 50µL of blank brain tissue homogenate supernatant was transferred to sample vials for mass spectrometry analysis. For actual brain tissue homogenate samples, 50µL of actual brain tissue homogenate supernatant was transferred to sample vials for mass spectrometry analysis.

Detection method and procedure: A QTRAP 6500 plus (SCIEX) mass spectrometer was used to determine the MS1 and MS2 information and corresponding mass spectrometric parameters of CKBA, thereby determining the quantitative ion pair. The CKBA standard was used as a methanol dilution (5µg/mL). The instrument used was a QTRAP 6500 plus (SCIEX). Mass spectrometry conditions comprised syringe pump direct injection (10µL/min), Q1 MS scan, positive ion mode, scan range: m/z 200-800, Gas1: 20; Gas2: 0; Curtain Gas: 20; Temperature: 0; ISVF: 5500; DP: 80, and/or Product Ion scan, positive ion mode, specified precursor ion. From the MS 1 results, the precursor ion was determined to be 581.6. Detection parameters comprised Gas1: 20; Gas2: 0; Curtain Gas: 20; Temperature: 0; ISVF: 5500; DP: 80; CE: 50.

### 5. Pull-down and Western blot assays for direct binding affinity between CKBA and MFE-2

CKBA pull-down assay was performed to detect the direct interaction between CKBA and MFE-2. Specifically, CKBA was labeled with Biotin. CKBA-biotin was added to the culture medium at a final concentration of 5µM for 6 hours. Cells were then washed, lysed, and incubated with Streptavidin magnetic beads under rotation at 4°C for 3 hours. After washing the beads 4 times, loading buffer was added, and the samples were boiled at 96°C for 5 minutes. Western blot (WB) was used to detect MFE-2 protein levels to assess the direct interaction between the small molecule compound and MFE-2.

The Western blot procedure comprised: fresh cells or tissue samples were immersed in lysis buffer and placed on ice for 10-15 minutes. After observing that the cells/tissues were fully lysed into a homogeneous turbid liquid, the samples were placed on ice for an additional 10 minutes. Centrifugation was performed at 12000 rpm for 10 minutes, and the supernatant was collected. Total protein concentration was determined using the BCA method. Subsequently, steps including loading, electrophoresis, membrane transfer, blocking, and incubation with primary and secondary antibodies were carried out. After developing with ECL chemiluminescent substrate, ImageJ was used to analyze protein content.

### 6. CCK-8 assay of CKBA-treated LPS-activated BV2 cells

The mouse microglial cell line BV2 was seeded into 96-well flat-bottom plates and cultured overnight to allow full cell adherence. BV2 cells were activated by stimulation with LPS. LPS (Sigma) stock solution (5 mg/mL) was added to the BV2 cell culture supernatant and mixed well. After 12 hours of activation, cell morphology was observed. Then, CKBA working solution prepared as described above was added to the cell culture medium at a final concentration of 5µM, and the cells were cultured for an additional 6-24 hours.

Subsequently, a CCK8 assay (Dojindo, Japan) was performed to measure cell viability. The cell culture medium was removed from the 96-well plate, and 100 µL of diluted CCK8 reaction solution was added to each well. The plate was protected from light. Incubation conditions: 37°C incubator for about 1-2 hours. After 1 hour of incubation, the plate was removed, and the colorimetric (orange) signal was detected using a multifunctional microplate reader (Tecan Infinite 200 pro, Austria). Depending on the signal intensity, the plate could be further incubated for 1.5/2 hours, and the signal was measured at multiple time points. The colorimetric intensity values were recorded, and subsequent statistical analysis and graphing were performed using SPSS.

### 7. Seahorse assay of CKBA-treated, LPS-stimulated BV2 cells

The specific methods for CKBA treatment and LPS activation of the BV2 cell line were as described above.

The main steps of the Seahorse assay were as follows: Seahorse XF Cell Mitochondrial Stress Analysis: Single cells from brain tissue were obtained using the aforementioned experimental methods. Microglia in brain tissue were collected by flow cytometry sorting. Primary microglia were seeded into Seahorse XF cell culture plates 4-7 days in advance. After the cells were fully adherent, the probe plate was hydrated with double-distilled water and placed in a CO₂-free 37°C incubator overnight. The next day, the calibration solution was replaced to further equilibrate and hydrate the probes. On the day of the experiment, the assay solution was prepared, and the corresponding substrates (glucose, pyruvate, glutamine, etc.) from the kit were added. The pH was adjusted to 7.4. Cells were washed three times with the assay solution, and the cell culture medium was replaced with the assay solution. The plate was placed in a CO₂-free 37°C incubator for 1 hour before loading onto the instrument. The detection drugs (Oligo, FCCP, ROT/AA) were loaded into drug addition ports A, B, and C of the probe plate. The instrument was then run, the probe plate hydration and calibration steps were performed, and then the hydrated plate was replaced with the probe plate for detection. OCR/ECAR values were recorded at different time points after drug addition. After recording, the cell plate was removed, and the number of microglia per well was counted under an inverted microscope. After normalizing OCR/ECAR to the cell number, intracellular mitochondrial stress levels were quantitatively analyzed.

### 8. Therapeutic regimen with CKBA oral formulation

Oral administration by gavage and/or with feed and/or oral tablets.

For evaluation of therapeutic efficacy in animal models, a 15mg/mL CKBA suspension (solvent: 0.5% sodium carboxymethylcellulose) was administered by oral gavage at a volume of 200µL (equivalent to a dose of 100mg/kg). The treatment was given once every other day for 5 consecutive months. Therapeutic efficacy was evaluated after the treatment period.

### 9. Mouse models of neuroinflammation and neurodegenerative diseases

(1) 5xFAD transgenic mice: These mice overexpress mutant human APP (695) carrying the Swedish (K670N, M671L), Florida (I716V) and London (V717I) familial Alzheimer's disease (FAD) mutations, as well as human PS1 carrying two FAD mutations (M146L and L286V). Expressions of both transgenes are driven by the mouse neuron-specific regulatory element Thy1 promoter to achieve overexpression in the brain. This line exhibits high APP expression, associated with a high load and accelerated accumulation of the 42-amino acid β-amyloid (Aβ-42) species. 5XFAD mice produce almost exclusively Aβ-42 and rapidly accumulate it in the brain.
(2) Preparation of MS mouse (EAE) model and evaluation of CKBA intervention efficacy

Female C57BL/6 mice (10 weeks old) were immunized with MOG35-55 emulsion. PTX was administered on the day of immunization and the following day. Twelve days after immunization, different doses of the small molecule drug CKBA were administered orally. The percentage change in body weight of each group was recorded, and the clinical manifestations of each group were scored. MOG35-55: myelin oligodendrocyte glycoprotein peptides 35 and 55. PTX: pertussis toxin. Fingolimod: Fingolimod.

### (3) Preparation of PD mouse model and evaluation of CKBA intervention efficacy

Healthy male SPF Balb/C mice, 4-6 weeks old, body weight 18-20 g, were intraperitoneally injected with MPTP at 20mg/kg/day for 14 consecutive days. The control group received intraperitoneal injections of an equal volume of physiological saline, with the same procedures and precautions. After MPTP administration, the model was considered successfully established. The corresponding CKBA drug or control solvent was administered. The normal group and PD model group received intraperitoneal injections of an equal volume of PBS, with the same procedures and precautions.

### (4) Evaluation of therapeutic efficacy in mouse models by behavioral tests (rotarod, water maze, open field)

Evaluation of the therapeutic effect of CKBA on AD: 8-10 month old 5xFAD mice, 5xFAD mice treated with the small molecule drug CKBA and control mice were subjected to behavioral tests including the Morris water maze, open field test and rotarod test to evaluate higher central nervous system functions. During the behavioral tests, the animal movement trajectory tracking system (EthoVision XT 16.0) was used for full-recording and offline analysis.

Morris water maze: Mice underwent a 5-day adaptive training period, wherein abnormal individuals were excluded. On the final day, after removing the platform, a probe trial was performed. Quantitative data were recorded, including the escape latency after entering the water in each quadrant during the learning phase, the number of platform crossings, and the latency to reach the platform area.

Open field test: The spontaneous activity path of mice in a 50cm*50cm*40 cm novel open field within 5 minutes was recorded. The distance traveled and time spent in the center area were analyzed to assess anxiety-related behavior.

Rotarod test: Mice were subjected to 3 adaptive training sessions, followed by consecutive tests of 5 minutes each. The latency to fall off the rotating rod was recorded to evaluate the motor function controlled by the nervous system.

### Example 1. Effect of MFE-2 knockout on pathological plaque deposition in the brain of AD animals

The inventors conditionally knocked out MFE-2 in microglia of AD mice (MFE-2^{cKO}5xFAD mice; referred to as ADcKO) (upper panel of Figure 1), and analyses were performed.

Behavioral experiments were conducted to compare the differences in higher central nervous system activities between the 5xFAD and MFE-2^{cKO}5xFAD mouse groups. As shown in the middle panel of Figure 1, it was found that the knockout group exhibited significantly reduced learning and memory abilities and a markedly increased average anxiety level compared with the control AD mice.

Further Aβ and microglial staining revealed that in the brains of MFE-2 conditional knockout AD mice, the morphology of microglia in the early stage was significantly abnormal, and the density of branched synaptic protrusions was decreased, suggesting impaired immune surveillance and neural support functions. By the time the mice survived to 8 months of age, the number of Aβ plaques in the hippocampal region of the brain was significantly increased, indicating that MFE-2 deficiency causes immune inflammatory dysfunction and exacerbates Aβ plaque deposition in brain tissue (lower panel of Figure 1).

Therefore, MFE-2 deficiency causes a significant decline in microglial function, leading to the progression of AD pathology and exacerbation of central nervous system damage, including impairment of learning and memory functions.

The above results demonstrate that MFE-2 knockout leads to a significant increase in pathological plaque deposition in the brain of AD animals, and that MFE-2 is a key therapeutic target for effectively inhibiting Aβ deposition.

### Example 2. Blood-brain barrier permeability of CKBA

Whether CKBA can effectively alleviate AD is an exploration of great potential clinical value. The inventors used mass spectrometry to evaluate the blood-brain barrier permeability of CKBA. First, an effective method for absolute quantification of CKBA by mass spectrometry was established. According to the optimized chromatographic and mass spectrometric methods described above, CKBA standard was analyzed. Initially, 10 ng/mL of CKBA was injected, and it was found that a characteristic chromatographic peak eluted at approximately 4.52min with a high response. When the concentration of the CKBA standard was reduced to 1ng/mL and further analyzed, a characteristic chromatographic peak was still observed at approximately 4.52min, and its response was about 1/10 of that of the 10ng/mL CKBA. The standard curve results showed good linearity and detection accuracy, indicating that the established quantitative method could accurately quantify the concentration of CKBA in methanol solution. The precursor ion obtained by MS1 scanning was 581.6, corresponding to CKBA plus one proton in the mass spectrometer. The corresponding product ion fragmentation spectrum was obtained by MS 2 scanning. The same quantitative product ion as that selected in previous company testing, 407.5, was chosen. Thus, the quantitative ion pair for CKBA was determined to be 581.6/407.5 (Figure 2A). According to Figure 2B, the established quantitative method for CKBA was specific, and the standard curve showed good linearity.

Based on the above optimized method, the absolute content of CKBA in brain tissue was further detected. For absolute content detection of CKBA in brain tissue, normal mice were administered CKBA by oral gavage. One hour after administration, the mice were perfused, and fresh brain tissue and serum were collected for mass spectrometry analysis.

The results showed that CKBA could effectively cross the blood-brain barrier (Figure 2C-E).

Therefore, the ability of CKBA to effectively cross the blood-brain barrier is an important basis for the systemic administration of CKBA in the treatment of neurodegenerative diseases.

### Example 3. CKBA directly binds to MFE-2 in living cells and protects the intracellular stability of MFE-2

CKBA (molecular formula: C37H56O5, molecular weight: 580.85, solubility: lipophilic, soluble in methanol/DMSO) (Figure 3A). 3-o-α-cyclohexanoyl-11-keto-β-boswellic acid (CKBA) is a small molecule compound obtained by structural modification and optimization using the active natural product molecule AKBA as a lead compound.

The inventors found that CKBA has a very strong affinity for MFE-2, with a KD of 0.97 nM (Figure 3B).

Through in vitro experiments and pull-down assays, it was shown that CKBA could bind to the MFE-2 protein in the microglial cell line (BV2) (Figure 3C).

Further exploration was conducted to investigate the mode of action of CKBA on the MFE-2 protein. The inventors treated normal BV2 cells with CKBA and found, by detecting protein expression, that CKBA did not affect the baseline expression level of MFE-2 in the cells. After LPS stimulation, the expression of MFE-2 in BV2 cells was significantly downregulated. Treatment with CKBA effectively maintained the expression and content of MFE-2 in the cells (Figure 3D-E).

Based on the above results, MFE-2 has an effective protective effect on the prognosis of AD and other neurodegenerative diseases, and CKBA can effectively maintain the expression of MFE-2 in cells.

### Example 4. Systemic administration of CKBA targeting MFE-2 effectively ameliorates the pathological process of neurodegenerative changes in AD mice and exerts therapeutic effects

### 1. Regulatory effect of CKBA targeting MFE-2 on microglial activation

Based on the previous findings that MFE-2 is closely associated with microglial activation in AD, in order to further clarify the regulatory effect of CKBA targeting MFE-2 on microglial activation, as well as its impact on AD central inflammation and brain functional prognosis, in vivo and in vitro CKBA intervention studies were conducted.

In vitro cell experiments were performed to analyze the effect of CKBA on the inflammatory activation of microglia. After 0.1-20 µM CKBA was added to LPS-activated BV2 cells for 24 hours, it was found that CKBA significantly inhibited the expansion of inflammatory microglia, maintained peroxisome stability, and exerted an anti-inflammatory effect (Figure 4A).

Seahorse energy metabolism analysis was performed. By measuring the changes in oxygen consumption reagents and dissolved oxygen content in the cell culture medium, the OCR of the cells was calculated. As shown in Figure 4B, CKBA significantly inhibited the activity of inflammatory microglia.

Therefore, CKBA, by targeting MFE-2, inhibits microglial inflammation and exerts an important therapeutic effect in suppressing neuroinflammation.

### 2. CKBA ameliorates disease symptoms in AD mice

AD mice were treated with CKBA by oral gavage (Figure 5A) for in vivo intervention studies, with continuous intervention for 3 months. The results showed that, compared with the vehicle control group, the CKBA-treated group exhibited a significant increase in platform crossover number, a significant decrease in time spent in the opposite quadrant, and a significant increase in time spent in the specific effective area (center area) as a percentage of total movement time. Therefore, CKBA significantly alleviated the decline in higher central nervous system functions such as learning and memory in AD mice (Figure 5B-C).

Thus, CKBA has great potential to inhibit central nervous system inflammation in AD and slow disease progression by targeting MFE-2.

The above results demonstrate that the CKBA compound can effectively improve the phenotype of central nervous system inflammation, and its therapeutic effect on central nervous system inflammation is surprising.

### Example 5. Synergistic effect of CKBA combined with donepezil on ameliorating central nervous system diseases in animals

In order to identify a substance that can be used in combination with CKBA to further enhance efficacy, the inventors conducted extensive research and screening efforts. It was found that donepezil could be used in combination with CKBA, exerting a significant synergistic effect.

AD mice were treated with CKBA (100mg/kg/day) combined with donepezil (3mg/kg/day) by oral gavage for in vivo intervention studies.

As shown in Figure 6, after continuous intervention for 3 months, it was shown that CKBA combined with donepezil significantly alleviated the degree of decline in higher central nervous system functions such as learning and memory in AD mice. A significant difference was observed between the male mouse model group and the normal group. The CKBA plus donepezil group showed a significant difference compared with the CKBA alone group and the donepezil alone group. Thus, the CKBA plus donepezil group had a superior ameliorative effect on the spatial exploration ability of the mice.

The results of the Morris water maze test showed that the learning and memory abilities of AD model animals were further and better improved by the combination of CKBA and donepezil.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. Use of a compound as shown in formula (I), or an isomer, solvate, prodrug, or pharmaceutically acceptable salt thereof, in the preparation of a pharmaceutical composition for relieving or treating central nervous inflammation; 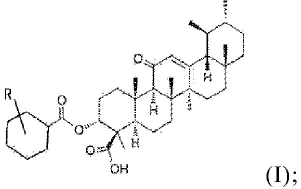 wherein R is independently selected from: hydrogen, hydroxy, C1-C4 alkyl, C2-C4 alkenyl, C2-C4 alkynyl, halogen.

2. The use according to claim 1, wherein the compound as shown in formula (I), or an isomer, solvate, prodrug, or pharmaceutically acceptable salt thereof, can target and bind to hydroxysteroid 17-β dehydrogenase 4, maintain the structural stability or activity of hydroxysteroid 17-β dehydrogenase and maintain the stability or activity of peroxisomes, thereby relieving or treating central nervous inflammation; or
the compound as shown in formula (I), or an isomer, solvate, prodrug, or pharmaceutically acceptable salt thereof, can target hydroxysteroid 17-β dehydrogenase 4, inhibit neuroglial cell overactivation related neuroinflammation, relieve neuron damage or death rate, or improve the cell metabolism microenvironment of the central nervous system.

3. The use according to claim 1, wherein, the central nervous inflammation is central nervous inflammation with dysfunction of hydroxysteroid 17-β dehydrogenase 4.

4. The use according to claim 1, wherein, the central nervous inflammation comprises: degenerative diseases of the central nervous system or chronic central inflammatory diseases.

5. The use according to claim 4, wherein, the degenerative diseases of the central nervous system comprise: Parkinson's disease, amyotrophic lateral sclerosis, Alzheimer's disease, Huntington's disease, or a disease of reduced learning ability or memory.

6. Use of a compound as shown in formula (I), or an isomer, solvate, prodrug, or pharmaceutically acceptable salt thereof, in the preparation of a pharmaceutical composition for maintaining the structural stability or activity of hydroxysteroid 17-β dehydrogenase 4, or maintaining the stability or activity of peroxisomes; 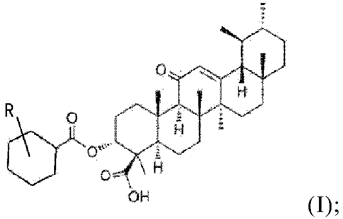 wherein R is independently selected from: hydrogen, hydroxy, C1-C4 alkyl, C2-C4 alkenyl, C2-C4 alkynyl, halogen.

7. Use of a compound as shown in formula (I), or an isomer, solvate, prodrug, or pharmaceutically acceptable salt thereof, in the preparation of a pharmaceutical composition for inhibiting neuroglial cell overactivation related neuroinflammation, relieving neuron damage or death rate, or improving the cell metabolism microenvironment of the central nervous system; 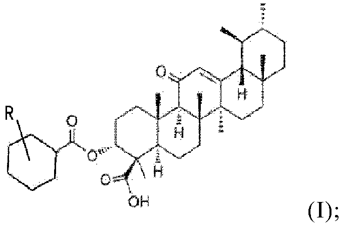 wherein R is independently selected from: hydrogen, hydroxy, C1-C4 alkyl, C2-C4 alkenyl, C2-C4 alkynyl, halogen.

8. The use according to claim 1, 6, or 7, wherein, the pharmaceutical composition further comprises donepezil.

9. The use according to claim 8, wherein, the ratio of the compound as shown in formula (I) to donepezil is from 10:1 to 60:1.

10. The use according to claim 9, wherein, the ratio of the compound as shown in formula (I) to donepezil is from 15:1 to 50:1.

11. The use according to claim 10, wherein, the ratio of the compound as shown in formula (I) to donepezil is from 20:1 to 45:1.

12. The use according to claim 1, 6, or 7, wherein, the compound as shown in formula (I) is a compound with the structure as shown in formula (II):

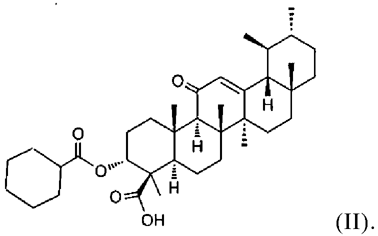

13. A composition for relieving or treating central nervous inflammation, comprising: a compound as shown in formula (I), or an isomer, solvate, prodrug, or pharmaceutically acceptable salt thereof; wherein the pharmaceutical composition further comprises donepezil; and wherein the ratio of the compound as shown in formula (I) to donepezil is from 10:1 to 60:1.

14. The composition according to claim 13, wherein, the ratio of the compound as shown in formula (I) to donepezil is from 15:1 to 50:1.

15. A kit for relieving or treating central nervous inflammation, comprising the composition according to claim 13 or 14.
